# EUROPÄISCHE PATENTSCHRIFT

(11) **EP 2 033 685 B1**
(45) Veröffentlichungstag und Bekanntmachung des Hinweises auf die Patenterteilung: **04.12.2019**
(21) Anmeldenummer: 08161340.8
(22) Anmeldetag: 29.07.2008
(51) Int. Cl.: A61N 1/372

(54) **FERNPROGRAMMIERBARES PERSÖNLICHES GERÄT SOWIE ANORDNUNG UND VERFAHREN ZUM FERNPROGRAMMIEREN EINES PERSÖNLICHEN GERÄTES**
REMOTE PROGRAMMABLE PERSONAL DEVICE AND DEVICE AND METHOD FOR PROGRAMMING A PERSONAL DEVICE REMOTELY
APPAREIL PERSONNEL TÉLÉCOMMANDÉ AINSI QU'AGENCEMENT ET PROCÉDÉ DE PROGRAMMATION À DISTANCE D'UN APPAREIL PERSONNEL

(30) Priorität: 10.09.2007 DE 102007043090
(43) Veröffentlichungstag der Anmeldung: 11.03.2009
(73) Patentinhaber: BIOTRONIK SE & Co. KG, 12359 Berlin (DE)
(72) Erfinder: Severin, Thomas, 12249 Berlin (DE)
(74) Vertreter: Keck, Hans-Georg

(56) Entgegenhaltungen:
- EP-A2- 1 048 323
- DE-A1- 10 053 116
- US-A- 5 456 692
- US-A1- 2003 009 203
- US-A1- 2007 185 547

## Beschreibung

Die Erfindung betrifft ein fernprogrammierbares persönliches Gerät, insbesondere ein programmierbares implantierbares medizinisches Gerät, beispielsweise einen Herzschrittmacher, einen Defibrillator, einen Kardioverter oder dergleichen. Außerdem betrifft die Erfindung eine Anordnung für die Fernprogrammierung eines solchen persönlichen medizinischen Gerätes und ein Verfahren zum Fernprogrammieren eines programmierbaren persönlichen Gerätes.

Im Zusammenhang mit Herzschrittmachern oder Defibrillatoren im Verbund mit einem Service Center ist es möglich und auch hinlänglich bekannt, seitens eines Herzschrittmachers oder Defibrillators gewonnene medizinische, physiologische oder Betriebsdaten zu dem zentralen Service Center zu übertragen, und diese Daten dort auszuwerten und über eine entsprechende Benutzerschnittstelle einem betreuenden Arzt zur Verfügung zu stellen.

Einige Funktionen solcher Implantate sind durch Software oder Firmware gesteuert und daher programmierbar. Hierzu weist ein solches Implantat eine programmierbare Steuerung auf.

Es kommt immer wieder vor, dass nach anfänglicher Programmierung kurz vor, während oder nach der Implantation des Implantats weitere Programmierungen oder Umprogrammierungen wünschenswert sind, um das Implantat besser auf möglicherweise inzwischen veränderte Gesundheitszustände eines Patienten einstellen zu können oder um die Leistungsfähigkeit des Implantats anderweitig zu erhöhen. Häufig geschieht ein derartiges Programmieren oder Umprogrammieren dadurch, dass ein Arzt zu einem jeweiligen Implantat mit Hilfe eines Programmiergerätes eine kurzreichweitige, drahtlose Datenverbindung herstellt und das Implantat im Angesicht des Patienten programmiert.

Eine Programmierung oder Umprogrammierung des Implantats kann jedoch grundsätzlich aus der Ferne, beispielsweise über das zentrale Service Center, geschehen. Hierzu kann eine Datenverbindung zwischen dem Service Center und einem Patientengerät hergestellt werden, welches sich üblicherweise in der Nähe eines Patienten befindet und eine bidirektionale Datenverbindung zwischen dem Implantat und dem Patientengerät herstellen kann. Die Verbindung zwischen dem Service Center und dem Patientengerät kann dabei drahtlos oder drahtgebunden beispielsweise über das Telefonnetz, ausgebildet sein.

Bei der Fernprogrammierung eines derartigen persönlichen Gerätes, wie es ein solches Implantat oder auch das Patientengerät ist, besteht grundsätzlich das Problem, dass die Laufzeit von aus der Ferne erteilten Programmieraufträgen (dies sind beispielsweise Datenpakete, die Steuerparameter für die programmierbare Steuerung des persönlichen Gerätes enthalten) nicht von vorne herein sicher abzuschätzen ist, so dass beispielsweise Programmieraufträge nicht unbedingt in der Reihenfolge bei dem persönlichen Gerät eintreffen, in der sie abgegeben wurden. Dies kann Fehlprogrammierungen des persönlichen Gerätes zur Folge haben, die insbesondere dann, wenn das persönliche Gerät beispielsweise ein Implantat wie Herzschrittmacher ist, fatale Folgen haben kann. Das Dokument US-A-2003/009203 offenbart den nächstliegenden Stand der Technik.

Als Beitrag zur Lösung dieses Problems wird erfindungsgemäß vorgeschlagen, dass das programmierbare persönliche Gerät wenigstens die Komponenten Datenkommunikations-Schnittstelle, Speicher und programmierbare Steuerung aufweist. Von diesen ist die Datenkommunikations-Schnittstelle so ausgebildet, dass das persönliche Gerät über die Datenkommunikations-Schnittstelle Programmieraufträge enthaltende Datenpakete empfangen kann. Der Speicher ist ausgebildet, wenigstens eine Auftragskennung zu speichern. Die programmierbare Steuerung ist ausgebildet, Funktionen des persönlichen Gerätes anhand von Steuerparametern zu steuern und mit der Datenkommunikations-Schnittstelle des persönlichen Gerätes wenigstens mittelbar verbunden. Die programmierbare Steuerung ist dazu ausgebildet, einen Programmierauftrag über die Datenkommunikations-Schnittstelle entgegen zu nehmen und dem Programmierauftrag eine Auftragskennung zu entnehmen und mit der gespeicherten Auftragskennung zu vergleichen. Weiterhin ist die programmierbare Steuerung ausgebildet, einen so empfangenen Programmierauftrag nur auszuführen, wenn die in einem jeweiligen Programmierauftrag enthaltende Auftragskennung der in dem Speicher des persönlichen Gerätes gespeicherten Auftragskennung entspricht. Andernfalls führt die programmierbare Steuerung den jeweils empfangenen Programmierauftrag nicht aus.

Bei einem derartigen persönlichen Gerät kann sichergestellt werden, dass nur ein Programmierauftrag bei dem persönlichen Gerät zur Ausführung kommen kann und damit zu einer Änderung der Steuerparameter der programmierbaren Steuerung des persönlichen Gerätes, wenn der Programmierauftrag die in dem persönlichen Gerät enthaltende Auftragskennung enthält. Um sicherzustellen, dass nicht nacheinander zwei Programmieraufträge, die die gleiche Auftragskennung enthalten, zur Ausführung kommen, ist die programmierbare Steuerung vorzugsweise weiterhin so ausgebildet, dass sie nach jedem Ausführen eines Programmierauftrags eine andere Auftragskennung in dem Speicher speichert.

Diese neue, nach Ausführung eines Programmierauftrags in dem persönlichen Gerät zu speichernde Auftragskennung kann entweder nach Ausführung eines Programmierauftrags in dem persönlichen Gerät selbst generiert werden, kann jedoch auch von jedem anderen von der Generierung, Übertragung oder Ausführung eines Programmierauftrags betroffenen Einheit, beispielsweise einem Service Center oder einem Patientengerät oder eine Programmiergerät erzeugt sein.

Gemäß einer Ausführungsvariante kann es vorgesehen sein, dass das persönliche Gerät eine zweite Datenkommunikationsschnittstelle neben der ersten Datenkommunikations-schnittstelle aufweist, wobei sich die zweite Datenkommunikationsschnittstelle von der ersten Datenkommunikationsschnittstelle hinsichtlich des erforderlichen Datenformats und/oder hinsichtlich der Funktechnik, auf der sie basiert, unterscheidet. Beispielsweise kann die erste Datenkommunikationsschnittstelle eine Schnittstelle für die Fernprogrammierung gemäß dem MICS-Standard sein, während die zweite Datenkommunikations-schnittstelle eine Schnittstelle für die Nahprogrammierung im Angesicht des Patienten mit Hilfe eines üblichen Programmiergerätes mit auf den Körper des Patienten auszulegendem Programmierkopf sein kann.

In diesem Fall ist es vorteilhaft, wenn die programmierbare Steuerung ausgebildet ist, einen Programmierauftrag ggf. über die zweite Datenkommunikations-Schnittstelle entgegenzunehmen und einen über die zweite Datenkommunikations-Schnittstelle empfangenen Programmierauftrag unabhängig davon auszuführen, ob der in dem über die zweite Datenkommunikations-Schnittstelle empfangene Programmierauftrag eine Auftragskennung enthält, die der gespeicherten Auftragskennung entspricht.

Dann kann das programmierbare persönliche Gerät - beispielsweise ein Herzschrittmacher - in jedem Fall und unabhängig vom Vorliegen miteinander konsistenter Auftragskennungen auf herkömmliche Art und Weise von einem Arzt mit Hilfe eines herkömmlichen Programmiergerätes im Angesicht des Patienten programmiert werden. Dies kann eine sinnvolle Fallback Variante darstellen.

In jedem Falle ist das persönliche Gerät so ausgebildet, dass es einen neuen Programmierauftrag überhaupt nur ausführen kann, wenn ein zuvor empfangener Programmierauftrag vollständig abgeschlossen ist.

Für den Fall das die programmierbare Steuerung des persönlichen Gerätes dazu ausgebildet ist, nach Abschluss eines Programmierauftrags eine neue Auftragskennung zu generieren und im eigenen Speicher zu speichern, ist das programmierbare persönliche Gerät vorzugsweise weiterhin dazu ausgebildet, diese neue Auftragskennung auch über eine Datenkommunikations-Schnittstelle an ein externes Gerät zu versenden. Nur dann ist es möglich, dass ein externes Gerät, welches an der Generierung und Übertragung eines neuen Programmierauftrags beteiligt ist, diese neue Auftragskennung einem jeweiligen Programmierauftrag hinzufügt.

Alternativ kann die programmierbare Steuerung auch ausgebildet sein, einen empfangenen und auszuführenden Programmierauftrag jeweils auch gleich eine neue Auftragskennung zu entnehmen. Diese neue Auftragskennung ist dann von einem externen Gerät neben der jeweils aktuellen, bereits im Speicher des persönlichen Geräts gespeicherten Auftragskennung einem jeweiligen Programmierauftrag beigefügt. In diesem Fall führt die programmierbare Steuerung einen jeweils empfangenen Programmierauftrag nur aus, wenn die in diesem Programmierauftrag enthaltende aktuelle Auftragskennung der in dem Speicher des persönlichen Gerät gespeicherten Auftragskennung entspricht. Nach Ausführen des Programmierauftrags oder während des Ausführen des Programmierauftrags entnimmt die programmierbare Steuerung dem jeweils auszuführenden oder ausgeführten Programmierauftrag die neue Auftragskennung und speichert diese in dem Speicher. Ein nachfolgender Programmierauftrag muss dann diese neue Auftragskennung enthalten, um ausgeführt werden zu können.

Vorteilhaft ist es, wenn das persönliche Gerät ausgebildet ist, nach erfolgreichem Ausführen eines Programmierauftrags einen Datensatz mit den nach Ausführen des Programmierauftrags aktuellen Steuerparametern für die Programmierbare Steuerung zusammen mit der neuen, in dem Speicher gespeicherten Auftragskennung an ein externes Gerät zu versenden. Damit liegen im Ergebnis in dem externen Gerät, beispielsweise dem Service-Center die jeweils aktuellen Steuerparameter sowie die neue Auftragskennung vor. Letztere kann wie nachfolgend beschrieben vorteilhaft für das Kennzeichnen von weiteren vom persönlichen Gerät versandten Daten genutzt werden.

Weiterhin ist die programmierbare Steuerung ausgebildet die zu einem zuletzt ausgeführten Programmierauftrag gehörende Auftragskennung oder die neue Auftragskennung solchen Daten hinzuzufügen, die von dem persönlichen Gerät nach dem zuletzt ausgeführten Programmierauftrag erfasst oder generiert wurden und die von der programmierbaren Steuerung an ein externes Gerät übertragen werden.

Auf diese Weise ist es möglich, dass allen Daten, die von einem externen Gerät seitens des persönlichen Gerätes empfangen werden, die genauen Steuerparameter zugeordnet werden können, auf deren Basis das persönliche Gerät beim Erfassen oder Generieren der ausgesendeten Daten operiert, da die Auftragskennung mit einem jeweiligen Programmierauftrag auch die in Folge des Programmierauftrags eingestellten Steuerparameterfür das persönliche Gerät kennzeichnet.

Das persönliche Gerät ist vorzugsweise ein aktives medizinisches Implantat und zwar bevorzugt ein implantierbarer Herzschrittmacher oder Defibrillator/Kardioverter. Insbesondere in diesem Falle ist bevorzugt, wenn die Datenkommunikations-Schnittstelle des persönlichen Gerätes für eine drahtlose Kommunikation mit einer Reichweite im Bereich von bis zu 5 m ausgebildet ist und insbesondere eine Datenkommunikations-Schnittstelle gemäß dem Medical Implant Communications Service Spezifikation (MICS) ist.

Wie zuvor erwähnt, wird das erfindungsgemäße persönliche Gerät vorzugsweise im Rahmen einer Anordnung zum Fernprogrammieren des programmierbaren persönlichen Gerätes eingesetzt. Diese Anordnung umfasst gemäß eines weiteren Aspektes der Erfindung neben dem persönlichen programmierbaren der vorbeschriebenen Art auch ein Programmiergerät zur Programmierung des persönlichen Gerätes aus der Ferne. Dieses Programmiergerät besitzt mindestens eine eigene Datenkommunikations-Schnittstelle zum wenigstens mittelbaren Verbinden des Programmiergerätes mit dem persönlichen Gerät sowie auch einen eigenen Speicher, der ausgebildet ist, wenigstens eine Auftragskennung zu speichern. Darüber hinaus besitzt das Programmiergerät eine Programmiereinheit für das persönliche Gerät, die ausgebildet ist, einen jeweiligen Programmierauftrag an dem Speicher gespeicherte Auftragskennung hinzuzufügen und die Übertragung des Programmierauftrages über die Datenkommunikations-Schnittstelle des Programmiergerätes zu veranlassen.

Dabei kann das Programmiergerät beispielsweise ein Programmiergerät im engeren Sinne sein, welches für die direkte Programmierung des persönlichen Gerätes in unmittelbarer Nähe beispielsweise eines Patienten ausgebildet ist. Das Programmiergerät kann auch von einem Service Center und einem mit dem Service Center verbundenen Endgerät gebildet sein. Das Programmgerät kann darüber hinaus auch wenigstens teilweise von einem so genannten Patientengerät gebildet sein, welches sich in bekannten Anordnung jeweils in der Nähe eines implantierbaren Implantats befindet und welches quasi als Relaisstation für die Übertragung von Daten oder Programmaufträgen zwischen einem Implantat und einem weiter entfernten Service Center dient, aber je nach Ausführung auch als selbständiges Programmiergerät neben einem herkömmlichen Programmiergerät dienen kann.

Gemäß einer Ausführungsvariante dieser Anordnung ist das Programmiergerät dazu ausgebildet, eine Auftragskennung über die Datenkommunikationsschnittstelle zu empfangen und in dem Speicher des Programmiergerätes zu speichern. Dies ist immer dann eine sinnvolle Ausführungsvariante, wenn eine jeweils neue Auftragskennung beispielsweise von dem programmierbaren persönlichen Gerät (also im speziellen Fall dem medizinischen Implantat) oder einem anderen Gerät in der Übertragungskette für Programmieraufträge generiert wird.

Für den Fall, dass eine neue Auftragskennung für einen zukünftigen Programmierauftrag nicht in dem programmierbaren persönlichen Gerät selbst gebildet wird, ist das Programmiergerät vorzugsweise ausgebildet, einem jeweiligen neuen Programmierauftrag neben einer aktuellen Auftragskennung die jeweilige neue Auftragskennung für einen zukünftigen Programmierauftrag hinzuzufügen. Die aktuelle Auftragskennung wird dazu benötigt, dass ein jeweiliger Programmierauftrag selbst überhaupt ausgeführt werden kann.

In diesem Fall ist das persönliche Gerät dazu ausgebildet, vor einem Ausführen eines jeweils empfangenen Programmierauftrags die in diesem Programmierauftrag enthaltende aktuelle Auftragskennung mit der im Speicher des persönlichen Gerätes gespeicherten Auftragskennung zu vergleichen und den jeweils empfangenen Programmierauftrag nur im Falle des Übereinstimmens auszuführen. Im Rahmen der Ausführung dieses Programmierauftrags oder nach Beendigung dieses Programmierauftrags speichert das persönliche Gerät die in dem Programmierauftrag enthaltende neue Auftragskennung in dem Speicher des persönlichen Gerätes als Auftragskennung für zukünftige Programmieraufträge.

Ein weiterer Beitrag zur Lösung des eingangs genannten Problems besteht in einem Verfahren zum Fernprogrammieren eines programmierbaren persönlichen Gerätes wie beispielsweise eines implantierbaren Herzschrittmachers oder Defibrillator/Kardioverters oder dergleichen, mit Hilfe der zuvor beschriebenen Anordnung. Dieses Verfahren umfasst die Verfahrensschritte des Anspruchs 13.

Weitere vorteilhafte Ausgestaltungen ergeben sich aus der Kombination der hier bereits genannten Merkmale und aus der nachfolgenden Beschreibung eines Ausführungsbeispiels.

Die Erfindung soll nun anhand eines solchen Ausführungsbeispiels mit Bezug auf Figuren näher erläutert werden. Diese zeigen in
- Fig. 1:: eine Anordnung zum Fernprogrammieren eines Implantats als persönliches Gerät, die ein derartiges persönliches Gerät enthält;
- Fig. 2:: eine detailliertere Darstellung der Anordnung aus Figur 1;
- Fig. 3:: den Ablauf einer erfolgreichen Fernprogrammierung;
- Fig. 4:: den Ablauf einer nicht erfolgreichen Programmierung; und
- Fig. 5:: eine Illustration der Zuordnung einer Auftragskennung zu vom persönlichen Gerät nach Außen gesandten Daten.

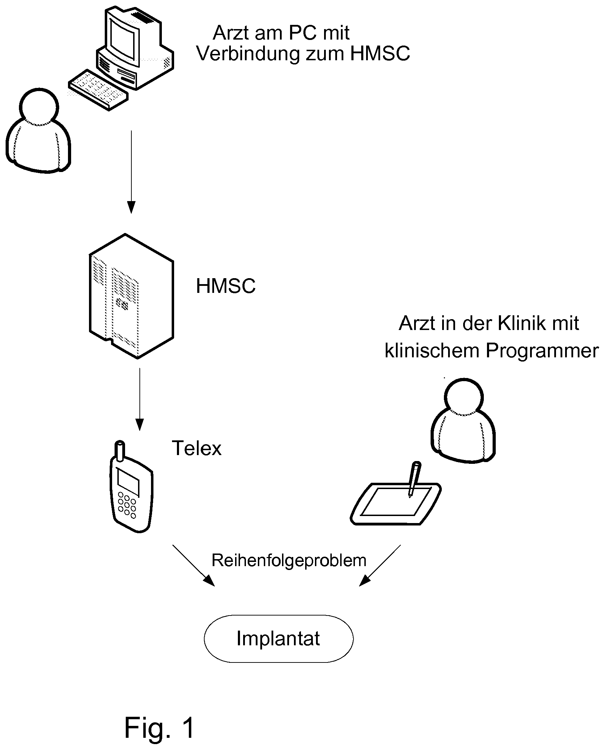

Figur 1 zeigt in einem Bild zwei mögliche Anordnungen zum Programmieren eines persönlichen Gerätes in Form eines Implantats 10.

Dieses Implantat 10 kann zum einen direkt mit Hilfe eines Programmiergerätes 20 programmiert werden. Das Programmiergerät 20 wird dazu in unmittelbare Nähe des Implantats 10 gebracht.

Darüber hinaus kann das Implantat 10 fernprogrammiert werden. Hierzu ist ein Patientengerät 30 vorgesehen, das sich üblicherweise in der Nähe des Implantats 10 befindet und das als Relaisstation für eine Datenverbindung mit einem zentralen Service Center 40 dient. Das zentrale Service Center 40 ist mit einem Endgerät 50, beispielsweise einem Computer eines Arztes verbunden. In dieser Konstellation bilden das Patientengerät 30, das Service Center 40 und das Endgerät 50 ebenfalls ein Programmiergerät im Sinne der Erfindung.

Wenn das Implantat 10 dem Stand der Technik entspricht, kann das Implantat 10 ohne weitere Einschränkungen sowohl über das Programmiergerät 20 als auch mit Hilfe des Service Centers 40 programmiert werden. Dabei kann es vorkommen, dass ein zuvor mit Hilfe des Service Centers 40 und des Endgerätes 50 erstellter Programmierauftrag wegen einer länger dauernden Übertragung des Programmierauftrags erst bei dem Implantat 10 eintrifft, nachdem zwischenzeitlich ein Arzt das Implantat 10 mit Hilfe des Programmiergerätes 20 direkt programmiert hat. Der zuletzt von dem Arzt mit Hilfe des Programmiergerätes 20 erstellte Programmierauftrag wird dann durch den zuvor mit Hilfe des Service Centers 40 und des Endgerätes 50 erstellten Programmierauftrag ersetzt. Der Arzt befindet sich jedoch in dem Irrglauben, sein zuletzt erstellter, jüngster Programmierauftrag sei in dem Implantat aktiv.

Um ein solches Szenario zu verhindern besitzt das erfindungsgemäße Implantat 10 neben einer Datenkommunikations-Schnittstelle 11 für die bidirektionale drahtlose Datenkommunikation mit dem Patientengerät 30 noch eine programmierbare Steuerung 13 und einen Speicher 15 für eine Auftragskennung. Die programmierbare Steuerung 13 ist dabei sowohl mit dem Speicher 15 als auch mit der Datenkommunikations-Schnittstelle 11 verbunden.

Gemäß einer bevorzugten Ausführungsvariante ist für die herkömmliche Programmierung des Implantats 10 mit einem üblichen Programmiergerät eine zweite Datenkommunikations-Schnittstelle 17 vorgesehen, über die das Implantat durch Auflegen eines Programmierkopfes eines Programmiergerätes programmiert werden kann.

Das Programmiergerät 20 besitzt eine Datenkommunikations-Schnittstelle 21 für eine direkte drahtlose Datenkommunikation mit dem Implantat 10. Mit dieser Datenkommunikations-Schnittstelle 21 ist eine Programmiereinheit 23 verbunden, die außerdem mit einem Speicher 25 zum Speichern einer Auftragskennung verbunden ist. Darüber hinaus besitzt das Programmiergerät 20 nicht weiter dargestellte Mittel zum Zusammenstellen eines Programmierauftrags. Die Programmiereinheit 23 ist dazu ausgebildet, einem jeweils zusammengestellten und abzusendenden Programmierauftrag 60 eine im Speicher 25 gespeicherte Auftragskennung hinzuzufügen.

Die programmierbare Steuerung 13 des Implantats ist dazu ausgebildet, einen über die erste Datenkommunikations-Schnittstelle 11 empfangenen Programmierauftrag 60 nur dann auszuführen, wenn die in ihm enthaltende Auftragskennung der in dem Speicher 15 des Implantats gespeicherten Auftragskennung entspricht.

Über die zweite Datenkommunikationsschnittstelle 17 kann das Implantat 10 mittels eines herkömmlichen Programmiergerätes z.B. durch Auflegen eines Programmierkopfes auch unabhängig von der im Implantat 10 gespeicherten Auftragskennung programmiert werden. Dies ist vorzugsweise nur dann möglich, wenn der Arzt über das Programmiergerät vor der Umprogrammierung das zuletzt eingestellte Programm gesehen hat und dies bestätigt hat. Vorzugsweise wird auch nach einer derartigen direkten Programmierung eine neue Auftragskennung für eine nachfolgende Fernprogrammierung generiert. Dies stellt sicher, dass eine möglicherweise bereits ausgelöste Fernprogrammierung mittels eines Programmierauftrags, der die ursprüngliche Auftragskennung enthält, der aber seitens des Implantats 10 zu dem Zeitpunkt, an dem die direkte Programmierung erfolgte, noch nicht empfangen wurde, nicht doch noch zur Wirkung kommen kann.

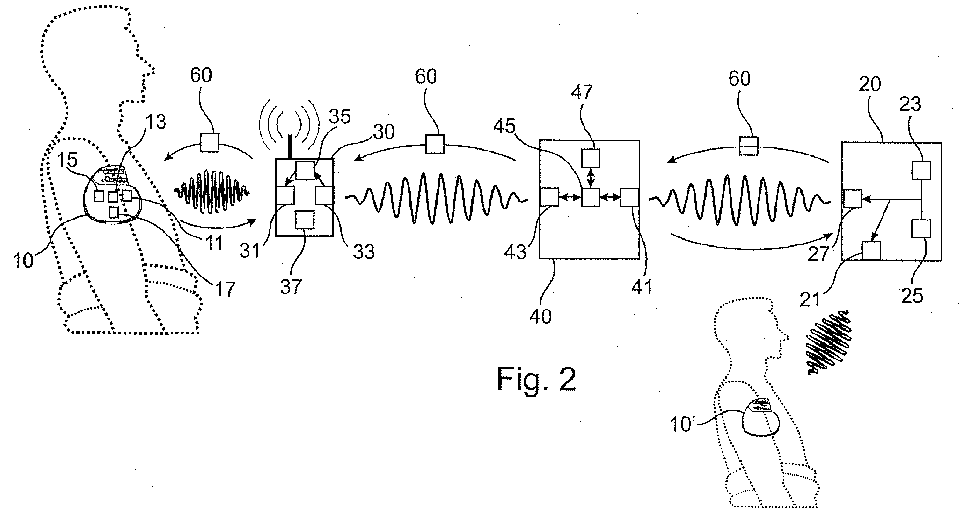

Anstelle mittels einer direkten drahtlosen Datenkommunikation zwischen dem Programmiergerät 20 und dem Implantat am Beispiel des Implantats 10' (wie in Figur 2 rechts unten gestrichelt dargestellt), kann die Programmierung des Implantats 10 auch aus der Ferne über das Patientengerät 30 und das Service Center 40 erfolgen. Dazu besitzt das Patientengerät 30 eine erste Datenkommunikations-Schnittstelle 31, die mit der Datenkommunikations-Schnittstelle 11 des Implantats 10 kompatibel ist. Außerdem besitzt das Patientengerät 30 eine zweite Datenkommunikations-Schnittstelle 33, über die das Patientengerät 30 eine Datenverbindung zu dem Service Center 40 herstellen kann. Die erste und die zweite Datenkommunikations-Schnittstelle 31 und 33 des Patientengerätes 30 sind mit einer Patientengerät-Steuerung 35 verbunden. Außerdem besitzt auch das Patientengerät 30 einen Speicher 37, der ebenfalls mit der Patientengerät-Steuerung 35 verbunden ist.

In analoger Weise kann das Service Center 40 eine erste Datenkommunikations-Schnittstelle 41 zur Anbindung eines Endgerätes z.B. eines Computers 50 (siehe Figur 1) oder aber auch zur Anbindung des Programmiergerätes 20 besitzen.

Das Programmiergerät 20 besitzt zu diesem Zweck eine zweite Datenkommunikations-Schnittstelle 27. Außerdem besitzt das Service Center 40 eine zweite Datenkommunikations-Schnittstelle 43, die mit der zweiten Datenkommunikations-Schnittstelle 33 des Patientengerätes 30 kompatibel ist. Auch das Service Center 40 besitzt eine Steuereinheit 45 und einen mit dieser verbundenen Speicher 47. Der Speicher 47 erlaubt es, auch in dem Service Center 40 eine Auftragskennung zu speichern. Wenigstens eines der Geräte Implantat 10, Patientengerät 30, Service Center 40 oder Programmiergerät 20 ist dazu ausgebildet, nach dem erfolgreichen Ausführen eins Programmierauftrages eine neue Auftragskennung zu generieren und wenigstens Implantat 10 und dem Programmiergerät 20 oder dem Service Center 40 zu übermitteln. Dabei ist jeweiliges Gerät dazu ausgebildet, die von ihm selbst generierte Auftragskennung oder eine von einem anderen Gerät empfangene Auftragskennung in dem jeweiligen Speicher für die Auftragskennung zu speichern. Bevorzugte Varianten des Generierens der Auftragskennung und des Speicherns einer neuen Auftragskennung sind eingangs bereits beschrieben.

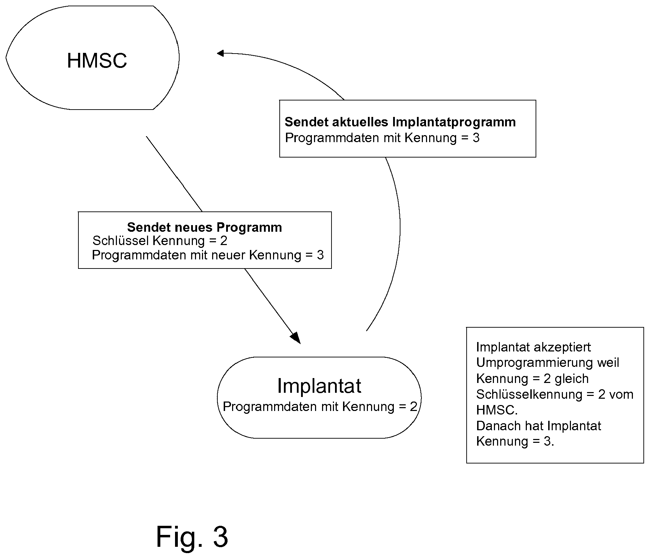

Figur 3 zeigt ein Beispiel, wie eine erfolgreiche Übertragung und Ausführung eines Programmierauftrages abläuft. In dem in Figur 3 dargestellten Beispiel stammt der Programmierauftrag 60 aus dem Service Center 40, welches den Programmierauftrag 60 versehen mit Parametern für die Einstellung der programmierbaren Steuerung 13 des Implantats 10 und einer Auftragskennung an das Implantat 10 überträgt. Im Beispiel ist die Auftragskennung eine numerische Ziffer, nämlich 2. Diese ist in dem Programmierauftrag 60 enthalten entspricht der in dem Speicher 15 des Implantats 10 gespeicherten Auftragskennung. Daher kann das Implantat 10 den Programmierauftrag 60 ausführen.

Der Programmierauftrag 60 enthält außerdem eine neue Auftragskennung für zukünftige Programmieraufträge. Diese entnimmt das Implantat 10 im Rahmen der Ausführung des Programmierauftrags 60 und speichert die neue Auftragskennung in seinem Speicher 15.

Zum Bestätigen der erfolgreichen Ausführung des Programmierauftrages übersendet das Implantat 10 anschließend die aktuell eingestellten Steuerparameter zusammen mit der neu gespeicherten Auftragskennung, die im vorliegenden Beispiel eine numerische Ziffer 3 ist.

Hier ist anzumerken, dass die Auftragskennung grundsätzlich ein beliebiges Datenformat haben kann und nicht unbedingt die Form von numerischen Ziffern.

Damit ist der Programmierauftrag erfolgreich abgeschlossen.

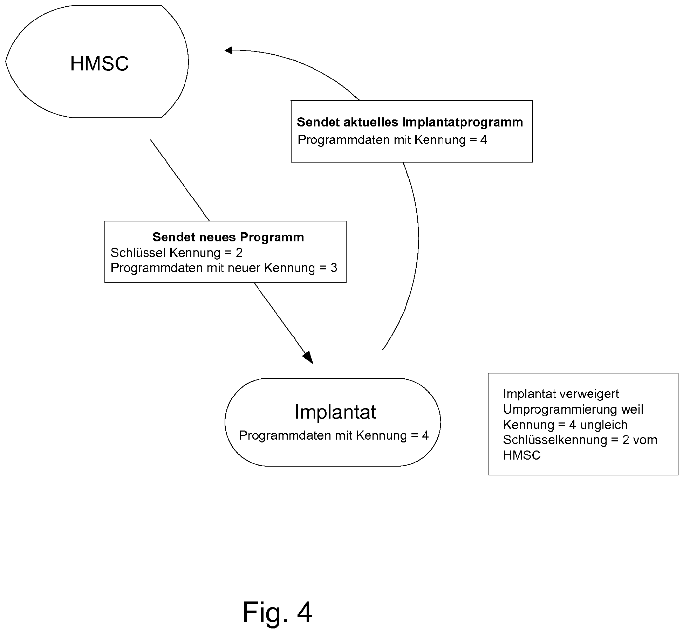

Figur 4 zeigt ein ähnliches Szenario wie Figur 3. Gemäß Figur 4 entspricht die im Implantat 10 gespeicherte Auftragskennung jedoch nicht die Auftragskennung, die ein vom Service Center 40 ausgesendeter Programmierauftrag 60 enthält. Daher führt das Implantat 10 den Programmierauftrag nicht aus.

In Reaktion auf den empfangenen, jedoch nicht ausgeführten Programmierauftrag 60 übersendet das Implantat 10 seine aktuellen Steuerparameter in Verbindung mit der in seinem Speicher 15 gespeicherten Auftragskennung, die in diesem Falle durch die Ziffer 4 gegeben ist.

Ähnlich wie das Implantat 10 nach Empfang beziehungsweise Ausführen eines jeweiligen Programmierauftrages die aktuellen Steuerparameter zusammen mit der aktuellen vorher gespeicherten Auftragskennung eines Service Center 40 übersendet, kann das Implantat 10 auch andere von ihm aufgenommene oder generierte Daten an das Service Center 40 übertragen. Solche Daten sind beispielsweise Daten zu einem intrakardial aufgenommenen EKG (IEGM) oder andere typischerweise von solchen Implantaten wie Herzschrittmachern oder Defibrillatoren zu einem Service Center übermittelte Daten. Gemäß der Erfindung fügt das Implantat 10 solchen an das Service Center 40 übersandten Daten jeweils die aktuell in seinem Speicher 40 gespeicherte Auftragskennung hinzu. Da das Implantat 10 zuvor mit dieser Auftragskennung verknüpfte aktuelle Steuerparameter an das Service Center 40 übermittelt hat, können alle weiteren von dem Implantat 10 an das Service Center 40 übermittelten Daten mittelbar diejenigen Steuerparameter zugeordnet werden, die die Funktion des Implantats 10 in dem Augenblick bestimmt haben, in dem das Implantat die übermittelten Daten erfasst oder generiert hat.

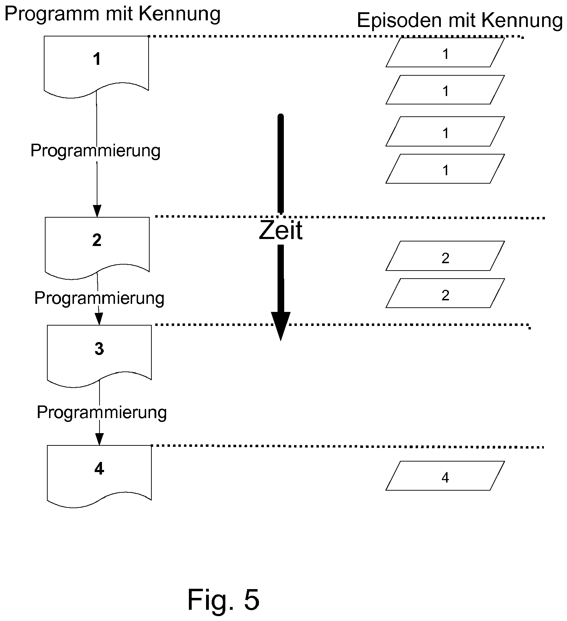

Diese Daten sind in Figur 5 als Episoden bezeichnet. Figur 5 stellt beispielhaft dar, wie die ein jeweiliges Programm definierenden Steuerparameter und die während der Ausführung eines jeweiligen Programms aufgezeichneten Episoden mit Hilfe der jeweils zu Grunde liegenden Auftragskennung leicht zugeordnet werden können.

## Patentansprüche

1. Programmierbares persönliches Gerät, insbesondere implantierbares medizinisches Gerät wie ein Herzschrittmacher, Defibrillator oder dergleichen, mit den Komponenten:
Datenkommunikations-Schnittstelle (11) die so ausgebildet ist, dass das persönliche Gerät über die Datenkommunikations-Schnittstelle Programmieraufträge (60) enthaltende Daten empfangen kann,
Speicher (15), der ausgebildet ist, wenigstens eine Auftragskennung zu speichern und programmierbare Steuerung (13) für Funktionen des persönlichen Gerätes anhand von Steuerparametern, die mit der Datenkommunikations-Schnittstelle wenigstens mittelbar verbunden und mindestens dazu ausgebildet ist, einen Programmierauftrag (60) über die Datenkommunikations-Schnittstelle (11) entgegenzunehmen und dem Programmierauftrag (60) eine Auftragskennung zu entnehmen und mit der gespeicherten Auftragskennung zu vergleichen und einen Programmierauftrag (60) nur auszuführen, wenn die in einem jeweiligen Programmierauftrag (60) enthaltene Auftragskennung der gespeicherten Auftragskennung entspricht und andernfalls den jeweils empfangenen Programmierauftrag (60) nicht auszuführen,
wobei die programmierbare Steuerung (13) dazu ausgebildet ist, nach Ausführen eines Programmierauftrags (60) eine neue Auftragskennung in dem Speicher (15) zu speichern und/oder eine neue Auftragskennung zu generieren und/oder die Übertragung einer neuen Auftragskennung über eine Datenkommunikations-Schnittstelle an ein externes Gerät (20; 30; 40) zu veranlassen; wobei die programmierbare Steuerung des Weiteren dazu ausgebildet ist, die zu einem zuletzt ausgeführten Programmierauftrag gehörende Auftragskennung oder die neue Auftragskennung solchen Daten hinzuzufügen, die von dem persönlichen Gerät nach dem zuletzt ausgeführten Programmierauftrag erfasst oder generiert wurden und die von der programmierbaren Steuerung an das externe Gerät (20, 30, 40) übertragen werden.

2. Programmierbares persönliches Gerät gemäß Anspruch 1, **dadurch gekennzeichnet, dass** die programmierbare Steuerung (13) dazu ausgebildet ist, die Übertragung der neuen Auftragskennung über die gleiche Datenkommunikations-Schnittstelle (11) zu veranlassen, die auch zum Empfangen von Programmieraufträgen ausgebildet ist und/oder einem auszuführenden Programmierauftrag (60) neben einer aktuellen eine zukünftige Auftragskennung zu entnehmen und zukünftige Auftragskennung nach Ausführen eines Programmierauftrags als neue Auftragskennung in dem Speicher (15) zu speichern.

3. Programmierbares persönliches Gerät gemäß einem der Ansprüche 1 oder 2, **dadurch gekennzeichnet, dass** das persönliche Gerät (10) ausgebildet ist, nach erfolgreichem Ausführen eines Programmierauftrags einen Datensatz mit den nach Ausführen des Programmierauftrags aktuellen Steuerparametern für die Programmierbare Steuerung zusammen mit der neuen, in dem Speicher gespeicherten Auftragskennung an ein externes Gerät zu versenden und/oder erfasste Daten oder Betriebsdaten an ein externes Gerät (20; 30; 40) zu übertragen und jeder derartigen Datenübertragung die in dem Speicher (15) gespeicherte Auftragskennung hinzuzufügen.

4. Programmierbares persönliches Gerät gemäß Anspruch 3, **dadurch gekennzeichnet, dass** das persönliche Gerät (10) ausgebildet ist, neben einer jeweiligen neuen Auftragskennung auch eine für die Ausführung eines jeweiligen jüngsten Programmierauftrags (60) verwendete Auftragskennung zu speichern und letztere und einer jeweiligen Datenübertragung von erfassten Daten oder Betriebsdaten hinzuzufügen und nur die neue Auftragskennung für den Vergleich mit einer Auftragskennung in einem eintreffenden Programmierauftrag heranzuziehen..

5. Programmierbares persönliches Gerät gemäß einem der Ansprüche 1 bis 4, **dadurch gekennzeichnet, dass** das persönliche Gerät ein aktives medizinisches Im-plantat (10) aus der Gruppe umfassend einen implantierbaren Herzschrittmacher (10), einen Defibrillator-Kardioverter und einen Neurostimulator.

6. Programmierbares persönliches Gerät gemäß einem der Ansprüche 1 bis 5, **dadurch gekennzeichnet, dass** die Datenkommunikations- Schnittstelle (11) des persönlichen Gerätes für eine drahtlose Kommunikation mit einer Reichweite im Bereich bis zu 5 m ausgebildet ist, insbesondere gemäß einer Medical Implant Communications Service Spezifikation (MICS).

7. Programmierbares persönliches Gerät gemäß einem der Ansprüche 1 bis 6, **dadurch gekennzeichnet, dass** das programmierbare persönliche Gerät eine zweite Datenkommunikationsschnittstelle (17) neben der ersten Datenkommunikationsschnittstelle (11) aufweist, wobei sich die zweite Datenkommunikationsschnittstelle (17) von der ersten Datenkommunikationsschnittstelle hinsichtlich des erforderlichen Datenformats und/oder hinsichtlich der Funktechnik , auf der sie basiert, unterscheidet.

8. Programmierbares persönliches Gerät gemäß Anspruch 7, **dadurch gekennzeichnet, dass** die programmierbare Steuerung (13) ausgebildet ist, einen Programmierauftrag (60) über die zweite Datenkommunikations-Schnittstelle (17) entgegenzunehmen und einen über die zweite Datenkommunikations-Schnittstelle (17) empfangenen Programmierauftrag (60) unabhängig davon auszuführen, ob der in dem über die zweite Datenkommunikations-Schnittstelle (17) empfangene Programmierauftrag (60) eine Auftragskennung enthält, die der gespeicherten Auftragskennung entspricht.

9. Anordnung zum Fernprogrammieren eines programmierbaren persönlichen Gerätes, insbesondere eines implantierbaren medizinischen Gerätes wie einem Herzschrittmacher, Defibrillator oder dergleichen, mit den Komponenten:
- persönliches programmierbares Gerät (10) gemäß einem der Ansprüche 1 bis 5 und
- Programmiergerät (20; 30, 40) zur Programmierung des persönlichen Gerätes (10) aus der Ferne,
wobei das Programmiergerät (20; 30, 40) wenigstens
eine Datenkommunikations-Schnittstelle (21; 31) zum wenigstens mittelbaren Verbinden des Programmiergerätes mit dem persönlichen Gerät,
einen Speicher, der ausgebildet ist, wenigstens eine Auftragskennung zu speichern und
eine Programmiereinheit (23) für das persönliche Gerät aufweist, die ausgebildet ist, einem Programmierauftrag eine Auftragskennung hinzuzufügen und dessen Übertragung über die Datenkommunikations-Schnittstelle zu veranlassen.

10. Anordnung nach Anspruch 9, **dadurch gekennzeichnet, dass** das Programmiergerät von einem Service-Center und einem mit dem Service-Center verbundenen Endgerät gebildet ist.

11. Anordnung nach Anspruch 9 oder 10, **dadurch gekennzeichnet, dass** das Programmiergerät ausgebildet ist, eine Auftragskennung über die Datenkommunikations-Schnittstelle (21; 31) zu empfangen und in dem Speicher (25; 47) des Programmiergerätes (20; 40) zu speichern.

12. Anordnung nach Anspruch 10 oder 11, **dadurch gekennzeichnet, dass** das Programmiergerät ausgebildet ist, einem Programmierauftrag neben einer aktuellen Auftragskennung eine neue Auftragskennung hinzuzufügen und dass das persönliche Gerät (10) ausgebildet ist vor einem Ausführen eines jeweils empfangenen Programmierauftrags (60) die aktuelle Auftragskennung mit der im Speicher des persönlichen Gerätes gespeicherten Auftragskennung zu vergleichen und nach Ausführen des Programmierauftrags die neue, in dem Programmierauftrag enthaltene Auftragskennung in dem Speicher des persönlichen Gerätes zu speichern.

13. Verfahren zum Fernprogrammieren eines programmierbaren persönlichen Gerätes, insbesondere eines implantierbaren medizinischen Gerätes wie einem Herzschrittmacher, Kardioverter/Defibrillator oder dergleichen, mittels einer Anordnung gemäß einem der Ansprüche 10 bis 13, mit den Verfahrensschritten:
- Auswählen eines zu programmierenden persönlichen Gerätes,
- Zusammenstellen eines Programmierauftrags für das ausgewählte persönliche Gerät,
- Hinzufügen einer Auftragskennung zu dem Programmierauftrag
- Übertragen des Programmierauftrags an das persönliche Gerät
- Empfangen des Programmierauftrags durch das persönliche Gerät
- Vergleichen der mit dem Programmierauftrag empfangenen Auftragskennung mit einer in dem persönlichen Gerät gespeicherten Auftragskennung, und
- entweder Ausführen des Programmierauftrags, wenn die mit dem Programmierauftrag empfangene Auftragskennung und die in dem persönlichen Gerät gespeicherte Auftragskennung identisch sind,
oder Nicht-Ausführen des Programmierauftrags, wenn die mit dem Programmierauftrag empfangene Auftragskennung und die in dem persönlichen Gerät gespeicherte Auftragskennung nicht miteinander übereinstimmen,
**gekennzeichnet durch** die zusätzlichen Verfahrensschritte:
- Generieren einer neuen Auftragskennung nach Ausführen eines Programmierauftrags,
- Speichern der neuen Auftragskennung in dem persönlichen Gerät, und
- Übertragen der neuen Auftragskennung an das Programmiergerät und
- Übertragen eines Datensatzes aktueller Steuerparameter und einer neuen Auftragskennung vom persönlichen Gerät an ein externes Gerät nach erfolgreichem Ausführen eines Programmierauftrags,
- Hinzufügen der zu einem zuletzt ausgeführten Programmierauftrag gehörenden Auftragskennung oder der neuen Auftragskennung zu solchen Daten, die von dem persönlichen Gerät nach dem zuletzt ausgeführten Programmierauftrag erfasst oder generiert wurden und an das externe Gerät übertragen werden.

## Claims

1. A programmable personal device, in particular an implantable medical device, such as a cardiac pacemaker, defibrillator or the like, comprising the components:
a data communication interface (11), which is designed such that the personal device may receive data containing programming instructions (60) via the data communication interface;
a memory (15), which is designed to store at least one instruction identifier; and
a programmable controller (13) for controlling functions of the personal device on the basis of control parameters, which programmable controller is at least indirectly connected to the data communication interface and is designed at least to accept a programming instruction (60) via the data communication interface (11) and to extract an instruction identifier from the programming instruction (60) and compare it to the stored instruction identifier and only to execute a programming instruction (60) if the instruction identifier contained in a particular programming instruction (60) corresponds to the stored instruction identifier and otherwise not to execute the particular programming instruction (60) received,
wherein the programmable controller (13) is designed, after execution of a programming instruction (60), to store a new instruction identifier in the memory (15) and/or to generate a new instruction identifier and/or to cause the transmission of a new instruction identifier via a data communication interface to an external device (20; 30; 40); wherein the programmable controller is also designed to append the instruction identifier belonging to a programming instruction last executed or to append the new instruction identifier to data that have been detected or generated by the personal device after the programming instruction last executed and that are transmitted from the programmable controller to the external device (20, 30, 40).

2. The programmable personal device according to claim 1, **characterised in that** the programmable controller (13) is designed to cause the transmission of the new instruction identifier via the same data communication interface (11) which is also designed to receive programming instructions and/or to extract a future instruction identifier in addition to a current instruction identifier from a programming instruction (60) to be executed and to store the future instruction identifier as new instruction identifier in the memory (15) after execution of a programming instruction.

3. The programmable personal device according to either one of claims 1 or 2, **characterised in that** the personal device (10) is designed, after successful execution of the programming instruction, to transmit a data set having the current control parameters for the programmable controller after execution of the programming instruction together with the new instruction identifier stored in the memory to an external device and/or to transmit acquired data or operational data to an external device (20; 30; 40) and to append the instruction identifier stored in the memory (15) to every data transmission of this type.

4. The programmable personal device according to claim 3, **characterised in that** the personal device (10) is implemented to store, in addition to a particular new instruction identifier, also an instruction identifier used for execution of a particular most recent programming instruction (60) and to append the latter and a particular data transmission of the acquired data or operational data and only to use the new instruction identifier for comparison to an instruction identifier in an incoming programming instruction.

5. The programmable personal device according to any one of claims 1 to 4, **characterised in that** the personal device is an active medical implant (10) from the group comprising an implantable cardiac pacemaker (10), a defibrillator-cardioverter, and a neurostimulator.

6. The programmable personal device according to any one of claims 1 to 5, **characterised in that** the data communication interface (11) of the personal device is implemented for wireless communication with a range up to 5 m, in particular according to a medical implant communications service specification (MICS).

7. The programmable personal device according to any one of claims 1 to 6, **characterised in that** the programmable personal device has a second data communication interface (17) in addition to the first data communication interface (11), wherein the second data communication interface (17) differs from the first data communication interface in regard to the required data format and/or in regard to the radio technology on which it is based.

8. The programmable personal device according to claim 7, **characterised in that** the programmable controller (13) is designed to accept a programming instruction (60) via the second data communication interface (17) and to execute a programming instruction (60) received via the second data communication interface (17) independently of whether the programming instruction (60) received via the second data communication interface (17) contains an instruction identifier which corresponds to the stored instruction identifier.

9. An assembly for remotely programming a programmable personal device, in particular an implantable medical device, such as a cardiac pacemaker, defibrillator or the like, comprising the components:
- a personal programmable device (10) according to any one of claims 1 to 5, and
- a programming device (20; 30; 40) for programming the personal device (10) remotely,
wherein the programming device (20; 30, 40) at least comprises
a data communication interface (21; 31) for at least indirect connection of the programming device to the personal device,
a memory, which is designed to store at least one instruction identifier, and
a programming unit (23) for the personal device, which is designed to append an instruction identifier to the programming instruction and cause its transmission via a data communication interface.

10. The assembly according to claim 9, **characterised in that** the programming device is formed by a service centre and a terminal connected to the service centre.

11. The assembly according to claim 9 or 10, **characterised in that** the programming device is designed to receive an instruction identifier via the data communication interface (21; 31) and to store it in the memory (25; 47) of the programming device (20; 40).

12. The assembly according to claim 10 or 11, **characterised in that** the programming device is designed to append a new instruction identifier to the programming instruction in addition to a current instruction identifier, and **in that** the personal device (10) is designed, before execution of a particular received programming instruction (60), to compare the current instruction identifier to the instruction identifier stored in the memory of the personal device and, after execution of the programming instruction, to store the new instruction identifier contained in the programming instruction in the memory of the personal device.

13. A method for remotely programming a programmable personal device, in particular an implantable medical device, such as a cardiac pacemaker, cardioverter/defibrillator or the like, by means of an assembly according to any one of claims 10 to 13, with the method steps:
- selecting a personal device to be programmed;
- compiling a programming instruction for the selected personal device;
- appending an instruction identifier to the programming instruction;
- transmitting the programming instruction to the personal device;
- receiving the programming instruction by the personal device;
- comparing the instruction identifier received with the programming instruction to an instruction identifier stored in the personal device; and
- either executing the programming instruction, if the instruction identifier received with the programming instruction and the instruction identifier stored in the personal device are identical,
or not executing the programming instruction if the instruction identifier received with the programming instruction and the instruction identifier stored in the personal device do not correspond to one another,
**characterised by** the additional method steps:
- generating a new instruction identifier after executing a programming instruction;
- storing the new instruction identifier in the personal device; and
- transmitting the new instruction identifier to the programming device; and
- transmitting a data set of current control parameters and a new instruction identifier from the personal device to an external device after successful execution of a programming instruction,
- appending the instruction identifier belonging to a programming instruction last executed or appending the new instruction identifier to data that were detected or generated by the personal device after the programming instruction last executed and that are transmitted to the external device.

## Revendications

1. Appareil personnel programmable, notamment appareil médical implantable tel qu'un stimulateur cardiaque, un défibrillateur ou similaire, avec les composantes:
une interface de communication de données (11) qui est conçue de telle manière que l'appareil personnel peut recevoir des données contenues dans des ordres de programmation (60) par le biais de l'interface de communication de données,
une mémoire (15) qui est conçue pour stocker au moins un identifiant d'ordre, et
une commande (13) programmable pour des fonctions de l'appareil personnel à l'aide de paramètres de commande qui sont reliés au moins indirectement avec l'interface de communication de données et sont au moins conçus pour accepter un ordre de programmation (60) par le biais de l'interface de communication de données (11) et pour prélever un identifiant d'ordre de l'ordre de programmation (60) et pour comparer avec l'identifiant d'ordre stocké et n'effectuer un ordre de programmation (60) uniquement si l'identifiant d'ordre contenu dans un ordre de programmation (60) respectif correspond à l'identifiant d'ordre stocké et dans le cas contraire, ne pas effectuer l'ordre de programmation (60) reçu respectif,
dans lequel la commande (13) programmable est conçue pour, après l'exécution d'un ordre de programmation (60), stocker un nouvel identifiant dans la mémoire (15) et/ou pour générer un nouvel identifiant d'ordre, et/ou pour provoquer la transmission d'un nouvel identifiant d'ordre à un appareil externe (20; 30; 40) par le biais d'une interface de communication de données ; où la commande programmable est en outre conçue pour ajouter à un dernier identifiant d'ordre faisant partie de l'ordre de programmation exécuté en dernier lieu ou au nouvel identifiant d'ordre des données qui ont été saisies ou générées par l'appareil personnel après l'ordre de programmation exécuté en dernier lieu et qui ont été transmises de la commande programmable à l'appareil externe (20, 30, 40).

2. Appareil personnel programmable selon la revendication 1, **caractérisé en ce que** la commande programmable (13) est conçue pour provoquer la transmission du nouvel identifiant d'ordre par le biais de la même interface de communication de données (11) qui est également conçue et/ou pour accepter un ordre de programmation (60) à exécuter à côté d'un identifiant d'ordre à venir actuel et enregistrer dans la mémoire (15) un identifiant d'ordre à venir après l'exécution d'un ordre de programmation servant d'identifiant d'ordre.

3. Appareil personnel programmable selon l'une des revendications 1 ou 2, **caractérisé en ce que** l'appareil personnel (10) est conçu, après l'exécution réussie d'un ordre de programmation, pour envoyer un ensemble de données avec les paramètres de commande actuels pour la commande programmable, conjointement avec le nouvel identifiant d'ordre stocké dans la mémoire, à un appareil externe et/ou transmettre des données détectées ou des données de fonctionnement à un appareil externe (20; 30; 40) et ajouter un identifiant d'ordre stocké dans la mémoire (15) à chaque transmission de données de ce type.

4. Appareil personnel programmable selon la revendication 3, **caractérisé en ce que** l'appareil personnel (10) est conçu pour, en plus d'un nouvel identifiant d'ordre respectif, stocker également un identifiant d'ordre utilisé pour l'exécution d'un ordre de programmation (60) respectivement plus récent et ajouter ce dernier et une transmission de données respective de données détectées ou de données de fonctionnement et ne faire appel au nouvel identifiant d'ordre que pour la comparaison avec un identifiant d'ordre dans un ordre de programmation entrant.

5. Appareil personnel programmable selon l'une des revendications 1 à 4, **caractérisé en ce que** l'appareil personnel est un implant médical actif (10) du groupe comprenant un stimulateur cardiaque (10) implantable, un défibrillateur-cardioverteur et un neurostimulateur.

6. Appareil personnel programmable selon l'une des revendications 1 à 5, **caractérisé en ce que** l'interface de communication de données (11) de l'appareil personnel est conçu pour une communication sans fil avec une portée dans la plage jusqu'à 5 m, notamment conformément à une spécification du service de communications d'implant médical (MICS).

7. Appareil personnel programmable selon l'une des revendications 1 à 6, **caractérisé en ce que** l'appareil personnel programmable présente une deuxième interface de communication de données (17) en plus de la première interface de communication (11), où la deuxième interface de communication de données (17) se distingue de la première interface de communication de données en ce qui concerne le format de données nécessaire, et/ou en ce qui concerne la technique de radiocommunication sur laquelle il est basé.

8. Appareil personnel programmable selon la revendication 7, **caractérisé en ce que** la commande programmable (13) est conçue pour accepter un ordre de programmation (60) par le biais de la deuxième interface de communication de données (17) et exécuter un ordre de programmation (60) reçu par le biais de la deuxième interface de communication de données (17) indépendamment du fait que l'ordre de programmation (60) reçu par le biais de la deuxième interface de communication de données (17) contient un identifiant d'ordre qui correspond à l'identifiant d'ordre stocké.

9. Ensemble de programmation à distance d'un appareil personnel programmable, notamment d'un appareil médical implantable tel qu'un stimulateur cardiaque, un défibrillateur ou similaire, avec les composantes:
- l'appareil personnel programmable (10) selon l'une des revendications 1 à 5, et
- un appareil de programmation (20; 30, 40) pour la programmation de l'appareil personnel (10) à distance,
dans lequel l'appareil de programmation (20; 30, 40) présente au moins
une interface de communication de données (21; 31) pour une connexion au moins indirecte de l'appareil de programmation avec l'appareil personnel,
une mémoire qui est conçue pour stocker au moins un identifiant d'ordre, et
une unité de programmation (23) pour l'appareil personnel qui est conçue pour ajouter un identifiant d'ordre à un ordre de programmation et provoquer sa transmission par le biais de l'interface de communication de données.

10. Ensemble selon la revendication 9, **caractérisé en ce que** l'appareil de programmation est formé par un appareil terminal d'un centre de service et est relié avec le centre de service.

11. Ensemble selon la revendication 9 ou la revendication 10, **caractérisé en ce que** l'appareil de programmation est conçu pour recevoir un identifiant d'ordre par le biais de l'interface de communication de données (21; 31) et pour le stocker dans la mémoire (25; 47) de l'appareil de programmation (20; 40).

12. Ensemble selon la revendication 10 ou la revendication 11, **caractérisé en ce que** l'appareil de programmation est conçu ajouter un nouvel identifiant d'ordre en plus d'un identifiant d'ordre actuel à un ordre de programmation et que l'appareil personnel (10) est conçu pour comparer l'identifiant d'ordre actuel avec l'identifiant d'ordre stocké dans la mémoire de l'appareil personnel avant une exécution d'un ordre de programmation (60) respectivement reçu, et après l'exécution de l'ordre de programmation, stocker le nouvel identifiant d'ordre contenu dans l'ordre de programmation dans la mémoire de l'appareil personnel.

13. Procédé de programmation à distance d'un appareil personnel programmable, notamment d'un appareil médical implantable tel qu'un stimulateur cardiaque, un cardioverteur/défibrillateur ou similaire, au moyen d'un ensemble selon l'une des revendications 10 à 13, avec les étapes de procédé:
- de sélection d'un appareil personnel programmable,
- d'assemblage d'un ordre de programmation pour l'appareil personnel programmable sélectionné,
- d'ajout d'un identifiant d'ordre à l'ordre de programmation
- de transmission d'un ordre de programmation à l'appareil personnel
- de réception de l'ordre de programmation par l'appareil personnel
- de comparaison de l'identifiant d'ordre reçu avec l'ordre de programmation avec un identifiant d'ordre stocké dans l'appareil personnel, et
- soit d'exécution de l'ordre de programmation si l'identifiant d'ordre reçu avec l'ordre de programmation et l'identifiant d'ordre stocké dans l'appareil personnel sont identiques,
soit de non exécution de l'ordre de programmation si l'identifiant d'ordre reçu avec l'ordre de programmation et l'identifiant d'ordre stocké dans l'appareil personnel ne correspondent pas l'un à l'autre,
**caractérisé par** les étapes de procédé complémentaires:
- de génération d'un nouvel identifiant d'ordre après l'exécution d'un ordre de programmation,
- de stockage du nouvel identifiant d'ordre dans l'appareil personnel, et
- de transmission du nouvel identifiant d'ordre à l'appareil de programmation, et
- de transmission d'un ensemble de données de paramètres de commande actuels et d'un nouvel identifiant d'ordre de l'appareil personnel vers un appareil externe après l'exécution réussie d'un ordre de programmation,
- d'ajout à un identifiant d'ordre faisant partie de l'ordre de programmation exécuté en dernier lieu ou au nouvel identifiant d'ordre des données qui ont été saisies ou générées par l'appareil personnel après l'ordre de programmation exécuté en dernier lieu et qui sont transmises à l'appareil externe.
